# EUROPÄISCHE PATENTSCHRIFT

(11) **EP 0 796 042 B1**
(45) Veröffentlichungstag und Bekanntmachung des Hinweises auf die Patenterteilung: **03.02.1999**
(21) Anmeldenummer: 95941028.3
(22) Anmeldetag: 27.11.1995
(51) Int. Cl.: A01N 51/00, A01N 61/00

(54) **DERMAL APPLIZIERBARE FORMULIERUNGEN VON PARASITIZIDEN**
PARASITICIDAL FORMULATIONS THAT CAN BE APPLIED DERMALLY
FORMULATIONS PARASITICIDES POUVANT ETRE APPLIQUEES SUR LA PEAU

(30) Priorität: 09.12.1994 DE 4443888
(43) Veröffentlichungstag der Anmeldung: 24.09.1997
(73) Patentinhaber: BAYER AG, 51368 Leverkusen (DE)
(72) Erfinder: SIRINYAN, Kirkor, D-51467 Bergisch Gladbach (DE); DORN, Hubert, D-42115 Wuppertal (DE); KUJANEK, Richard, D-51061 Köln (DE); KRIEGER, Klemens, D-51789 Lindlar (DE); HEUKAMP, Ulrich, D-51515 Kürten (DE); HACKEMÜLLER, Doris, D-40221 Düsseldorf (DE); HOPKINS, Terence, Taborine, QLD 4270 (AU)
(86) Internationale Anmeldenummer: EP9504667
(87) Internationale Veröffentlichungsnummer: WO9617520

(56) Entgegenhaltungen:
- EP-A- 0 303 490
- EP-A- 0 433 909
- EP-A- 0 483 052
- EP-A- 0 590 425
- EP-A- 0 682 869
- WO-A-93/24002
- DE-A- 2 331 793
- GB-A- 802 111
- GB-A- 2 194 147
- GB-A- 2 236 250
- US-A- 4 415 563

## Beschreibung

Die vorliegende Erfindung betrifft Formulierungen zur dermalen Bekämpfung von parasitierenden Insekten an Tieren mittels Agonisten oder Antagonisten der nicotinergen Acetylcholinrezeptoren von Insekten.

Agonisten oder Antagonisten der nicotinergen Acetylcholinrezeptoren von Insekten sind bekannt. Zu ihnen gehören die Nicotinyl-Insektizide und ganz besonders die Chlornicotinyl-Insektizide.

Aus der PCT-Anmeldung WO 93/24 002 ist bekannt, daß sich bestimmte 1-[N-(Halo-3-pyridylmethyl)]-N-methylamino-1-alkylamino-2-nitroethylen-Derivate zur systemischen Anwendung gegen Flöhe bei Haustieren eignen. Als ungeeignet zur Bekämpfung der Flöhe an Haustieren wird nach WO 93/24 002 die nicht-systemische d.h. dermale Art der Anwendung dargestellt.

Es wurden nun neue Formulierungen zur dermalen Anwendung von Agonisten oder Antagonisten der nicotinergen Acetylcholinrezeptoren von Insekten gefunden, die sich besonders zur dermalen Bekämpfung parasitierender Insekten wie Flöhen, Läusen oder Fliegen an Tieren eignen.

Die erfindungsgemäßen Formulierungen haben folgende Zusammensetzung:
- Agonisten oder Antagonisten der nicotinergen Acetylcholinrezeptoren von Insekten in einer Konzentration von 1 bis 20 Gew.-% bezogen auf das Gesamtgewicht der Formulierung;
- Lösungsmittel aus der Gruppe Benzylalkohol oder gegebenenfalls substituierte Pyrrolidone in einer Konzentration von mindestens 20 Gew.-% bezogen auf das Gesamtgewicht der Formulierung;
- gegebenenfalls weitere Lösungsmittel aus der Gruppe cyclischer Carbonate oder Lactone in einer Konzentration von 5,0 bis zu 80 Gew.-% bezogen auf das Gesamtgewicht der Formulierung;
- gegebenenfalls weitere Hilfsmittel aus der Gruppe Verdickungsmittel, Spreitmittel, Farbstoffe, Antioxidantien, Treibmittel, Konservierungsstoffe, Haftmittel, Emulgatoren, in einer Konzentration von 0,025 bis zu 10 Gew.-% bezogen auf das Gesamtgewicht der Formulierung.

Agonisten oder Antanogisten der nicotinogen Acetylcholinrezeptoren von Insekten sind bekannt z.B. aus Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17 659, 91/4965; Französische Anmeldung Nr. 2 611 114; Brasilianische Anmeldung Nr. 88 03 621.

Auf die in diesen Publikationen beschriebenen Verbindungen und ihre Herstellung wird hiermit ausdrücklich Bezug genommen.

Diese Verbindungen lassen sich bevorzugt durch die allgemeine Formel (I) wiedergeben 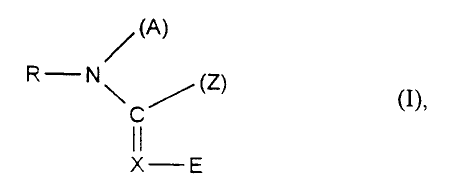 in welcher
- R: für Wasserstoff, gegebenenfalls substituierte Reste der Gruppe Acyl, Alkyl, Aryl, Aralkyl, Heteroaryl oder Heteroarylalkyl steht;
- A: für eine monofunktionelle Gruppe aus der Reihe Wasserstoff, Acyl, Alkyl, Aryl steht oder für eine bifunktionelle Gruppe steht, die mit dem Rest Z verknüpft ist;
- E: für einen elektronenziehenden Rest steht;
- X: für die Reste -CH= oder =N- steht, wobei der Rest -CH= anstelle eines H-Atoms mit dem Rest Z verknüpft sein kann;
- Z: für eine monofunktionelle Gruppe aus der Reihe Alkyl, -O-R, -S-R, 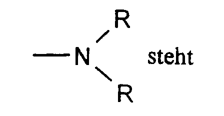 oder für eine bifunktionelle Gruppe steht, die mit dem Rest A oder dem Rest X verknüpft ist.

Besonders bevorzugt sind Verbindungen der Formel (I), in welcher die Reste folgende Bedeutung haben:
- R: steht für Wasserstoff sowie für gegebenenfalls substituierte Reste aus der Reihe Acyl, Alkyl, Aryl, Aralkyl, Heteroaryl, Heteroarylalkyl.
Als Acylreste seien genannt Formyl, Alkylcarbonyl, Arylcarbonyl, Alkylsulfonyl, Arylsulfonyl, (Alkyl-)-(Aryl)-phosphoryl, die ihrerseits substituiert sein können.
Als Alkyl seien genannt C₁₋₁₀-Alkyl, insbesondere C₁₋₄-Alkyl, im einzelnen Methyl, Ethyl, i-Propyl, sec.- oder t.-Butyl, die ihrerseits substituiert sein können.
Als Aryl seien genannt Phenyl, Naphthyl, insbesondere Phenyl.
Als Aralkyl seien genannt Phenylmethyl, Phenethyl,
Als Heteroaryl seien genannt Heteroaryl mit bis zu 10 Ringatomen und N, O, S insbesondere N als Heteroatomen. Im einzelnen seien genannt Thienyl, Furyl, Thiazolyl, Imidazolyl, Pyridyl, Benzthiazolyl.
Als Heteroarylalkyl seien genannt Heteroarylmethyl, Heteroarylethyl mit bis zu 6 Ringatomen und N, O, S, insbesondere N als Heteroatomen.
Als Substituenten seien beispielhaft und vorzugsweise aufgeführt: Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo (-SO₃H); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.
- A: steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O, S unterbrochen sein können.
- A und Z: können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Hexahydro-1,3,5-triazin, Morpholin genannt, die gegebenenfalls bevorzugt durch Methyl substituiert sein können.
- E: steht für einen elektronentziehenden Rest, wobei insbesondere NO₂, CN, Halogenalkylcarbonyl wie 1,5-Halogen-C₁₋₄-carbonyl, insbesondere COCF₃ genannt seien.
- X: steht für -CH= oder -N=
- Z: steht für gegebenenfalls substituierte Reste Alkyl, -OR, -SR, -NRR, wobei R und die Substituenten bevorzugt die oben angegebene Bedeutung haben.
- Z: kann außer dem obengenannten Ring gemeinsam mit dem Atom, an welches es gebunden ist und dem Rest 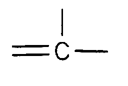 an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.
Als Beispiele für den heterocyclischen Ring seien Pyrrolidin, Piperidin, Piperazin, Hexamethylenimin, Morpholin und N-Methylpiperazin genannt.

Als ganz besonders bevorzugt erfindungsgemäß verwendbare Verbindungen seien Verbindungen der allgemeinen Formeln (II) und (III) genannt: 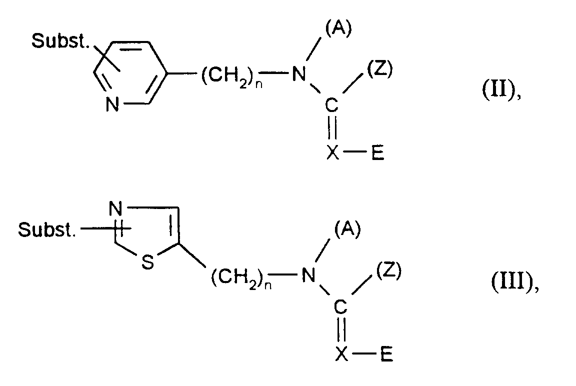 in welchen
- n: für 1 oder 2 steht,
- Subst.: für einen der oben aufgeführten Substituenten, insbesonders für Halogen, ganz besonders für Chlor, steht,
- A, Z, X und E: die oben angegebenen Bedeutungen haben,

Im einzelnen seien folgende Verbindungen genannt:

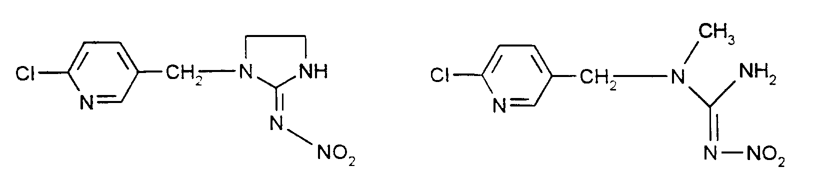

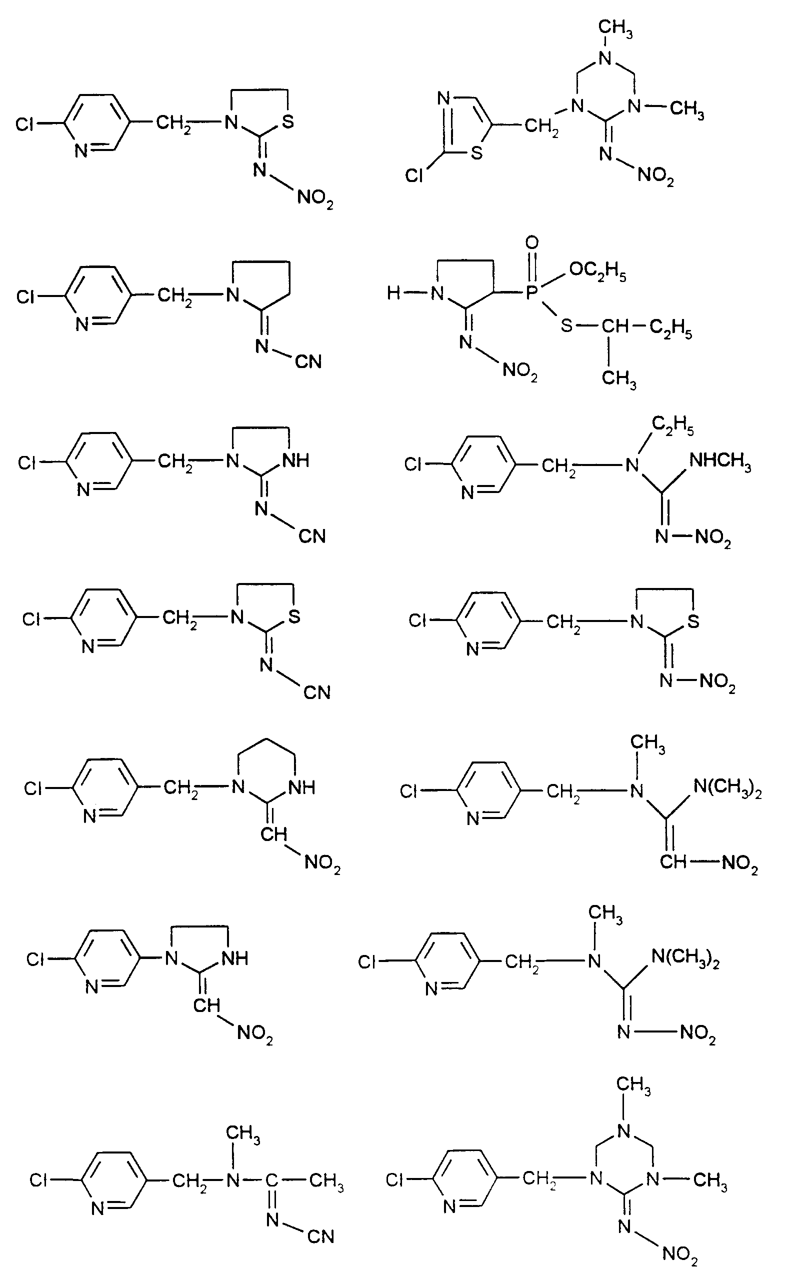

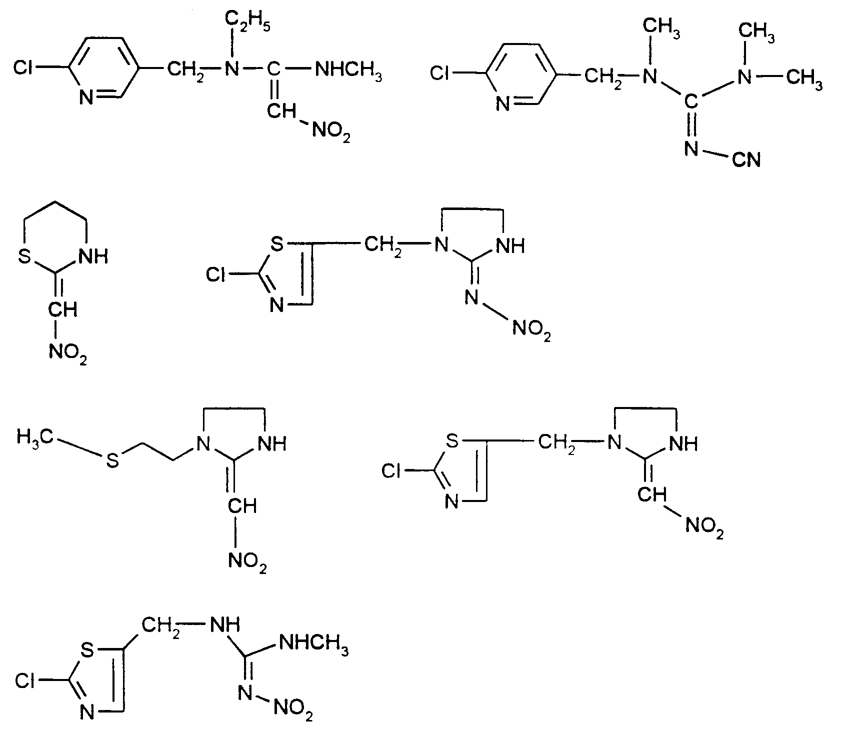

Die erfindungsgemäßen Formulierungen enthalten den Wirkstoff in Konzentrationen von 0,1 bis 20 Gew.-%, bevorzugt von 1 bis 12,5 Gew.-%.

Zubereitungen die vor Anwendung verdünnt werden, enthalten den Wirkstoff in Konzentrationen von 0,5 bis 90 Gew.-%, bevorzugt von 1 bis 50 Gew.-%.

Im allgemeinen hat es sich als vorteilhaft erwiesen, Mengen von etwa 0,5 bis etwa 50 mg, bevorzugt 1 bis 20 mg, Wirkstoff je Körpergewicht pro Tag zur Erzielung wirksamer Ergebnisse zu verabreichen.

Als Lösungsmittel kommen in Frage:
Benzylalkohol oder gegebenenfalls substituierte Pyrrolidone wie Pyrrolidon-2, 1-(C₂₋₂₀-Alkyl)-pyrrolidon-2, insbesondere 1-Ethylpyrrolidon, 1-Octylpyrrolidon, 1-Dodecylpyrrolidon, 1-Isopropylpyrrolidon, 1-(s. oder t. oder n-Butyl)-pyrrolidon, 1-Hexylpyrrolidon, 1-(C₂₋₁₀-Alkenyl)-pyrrolidon-2 wie 1-Vinylpyrrolidon-2, 1-(C₃₋₈-Cycloalkyl)-pyrrolidon-2 wie 1-Cyclohexylpyrrolidon, 1-(C₁₋₆-Hydroxyalkyl)-pyrrolidon-2, 1-(C₁₋₆-Alkoxy-C₁₋₆-alkyl)-pyrrolidon-2 wie 1-(2-Hydroxyethyl)-pyrrolidon, 1-(3-Hydroxypropyl)-pyrrolidon, 1-(2-Methoxyethyl)-pyrrolidon, 1-(3-Methoxypropyl)-pyrrolidon, ferner 1-Benzylpyrrolidon. Besonders genannt seien Benzylalkohol oder n-Dodecyl- oder n-Octylpyrrolidon. Diese Lösungsmittel können entweder allein oder in Mischung mit weiteren Lösungsmitteln (Colösungsmitteln) eingesetzt werden.

Sie liegen vor in einer Konzentration von mindestens 20 Gew.-%, bevorzugt 40 bis 90 Gew.-%, besonders bevorzugt 50 bis 90 Gew.-%.

Als weitere Lösungsmittel oder Colösungsmittel kommen in Frage: cyclische Carbonate oder Lactone. Als solche seien genannt: Ethylencarbonat, Propylencarbonat, γ-Butyrolacton.

Sie liegen vor in einer Konzentration von 5,0 bis zu 80 Gew.-%, bevorzugt von 7,5 bis 50 Gew.-%, besonders bevorzugt von 10 bis 50 Gew.-%.

Als weitere Hilfsmittel kommen in Frage: Konservierungsmittel wie Benzylalkohol (nicht erforderlich falls bereits als Lösungsmittel vorhanden), Trichlorbutanol, p-Hydroxybenzoesäureester, n-Butanol.

Verdickungsmittel wie: Anorganische Verdickungsmittel wie Bentonite, kolloidale Kieselsäure, Aluminiummonostearat, organische Verdickungsmittel wie Cellulosederivate, Polyvinylalkohole, Polyvinylpyrrolidone und deren Copolymere, Acrylate und Methacrylate.

Als Farbstoffe seien genannt alle zur Anwendung am Tier zugelassenen Farbstoffe, die gelöst oder suspendiert sein können.

Hilfsstoffe sind auch spreitende Öle wie Adipinsäure-di-2-ethylhexylester, Isopropylmyristat, Dipropylenglykolpelargonat, cyclische und acyclische Silikonöle wie Dimetikone und ferner deren Co- und Terpolymerisate mit Ethylenoxid, Propylenoxid und Formalin, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Hilfsstoffe sind auch Emulgatoren wie nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphor-säureester-monoethanolaminsalz;
   kationaktive Tenside wie Cetyltrimethylammoniumchlorid.

Weitere Hilfsstoffe sind Mittel mit denen die erfindungsgemäßen Formulierungen auf die Haut gesprüht oder gespritzt werden können. Dabei handelt es sich um die für Spraydosen benötigten üblichen Treibgase wie Propan, Butan, Dimethylether, CO₂ oder halogenierte Niedrigalkane, bzw. deren Mischungen untereinander.

Die erfindungsgemäßen Formulierungen eignen sich bei günstiger Warmblütertoxizität zur Bekämpfung von parasitierenden Insekten, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Besonders hervorgehoben sei die Wirkung gegen Siphonaptera, insbesondere gegen Flöhe.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

In den erfindungsgemäßen Formkörpern können auch weitere Wirkstoffe enthalten sein. Zu den weiteren Wirkstoffen gehören Insektizide wie phosphorhaltige Verbindungen, d.h. Phosphor- oder Phosphonsäureester, natürliche oder synthetische Pyrethroide, Carbamate, Amidine, Juvenilhormone und juvenoide synthetische Wirkstoffe, sowie Chitinsynthesehemmer wie Diarylether und Benzoylharnstoffe.

Zu den Phosphor- oder Phosphorsäureestern gehören:
0-Ethyl-0-(8-chinolyl)phenyl-thiophosphat (Quintiofos),
0,0-Diethyl-0-(3-chloro-4-methyl-7-coumarinyl)-thiophosphat (Coumaphos),
0,0-Diethyl-0-phenylglyoxylonitril-oxim-thiophosphat (Phoxim),
0,0-Diethyl-0-cyanochlorbenzaldoxim-thiophosphat (Chlorphoxim),
0,0-Diethyl-0-(4-bromo-2,5-dichlorophenyl)-phosphorothionat (Bromophos-ethyl),
0,0,0',0'-Tetraethyl-S,S'-methylene-di(phosphorodithionat) (Ethion),
2,3-p-Dioxanedithiol-S,S-bis(0,0-diethylphosphorodithionat,
2-Chlor-1-(2,4-dichlorphenyl)-vinyldiethylphosphat (Chlorfenvinphos),
0,0-Dimethyl-0-(3-methyl-4-methylthiophenyl)-thionophosphorsäureester (Fenthion).

Zu den Carbamaten gehören:
2-Isopropoxyphenylmethylcarbamat (Propoxur),
1-Naphthyl-N-methylcarbamat (Carbaryl).

Zu den synthetischen Pyrethroiden zählen
3-[2-(4-Chlorphenyl)-2-chlorvinyl]-2,2-dimethyl-cyclo-propancarbonsäure [(α-cyano-4-fluor-3-phenoxy)-benzyl]-ester (Flumethrin),
2,2-Dimethyl-3-(2,2-dichlorvinyl)-cyclopropancarbonsäure-α-cyano(4-fluor-3-phenoxy)-benzyl-ester (Cyfluthrin) und seine Enantiomere und Stereomere,
α-Cyano-3-phenoxybenzyl(±)-cis, trans-3-(2,2-dibromvinyl)-2,2-dimethylcyclopropancarboxylat (Deltamethrin),
2,2-Dimethyl-3-(2,2-dichlorvinyl)-cyclopropancarbonsäure-α-cyano-3-phenoxybenzylester (Cypermethrin),
3-Phenoxybenzyl(±)-cis, trans-3-(2,2-dichlorvinyl)-2,2-dimethylcyclopropancarboxylat (Permethrin),
α-(p-Cl-phenyl)-isovaleriansäure-α-cyano-3-phenoxy-benzylester (Fenvalerate),
2-Cyano-3-phenoxybenzyl-2-(2-chlor-α,α,α-trifluor-p-toluidino)-3-methylbutyrat (Fluvalinate).

Zu den Amidinen gehören:
3-Methyl-2-[2,4-dimethyl-phenylimino]-thiazolin,
2-(4-Chlor-2-methylphenylimino)-3-methylthiazolidin,
2-(4-Chlor-2-methylphenylimino)-3-(isobutyl-1-enyl)-thiazolidin
1,5-Bis-(2,4-dimethylphenyl)-3-methyl-1,3,5-triazapenta-1,4-dien (Amitraz).

Cyclische Makrolithe wie Ivermectine und Abamectine. Hierzu sei beispielsweise 5-0-Dimethyl-22,23-dihydroavermecti-A₁ₐ, -22,23-dihydroavermectin B₁ₐ und 22,23-dihydroavermectin B_{b1} (vgl. beispielsweise WHO. F.A. Series 27, S. 27-73 (1991)) erwähnt. Zu den Juvenilhormonen und juvenilhormonartigen Substanzen gehören insbesondere Verbindungen der folgenden Formeln:

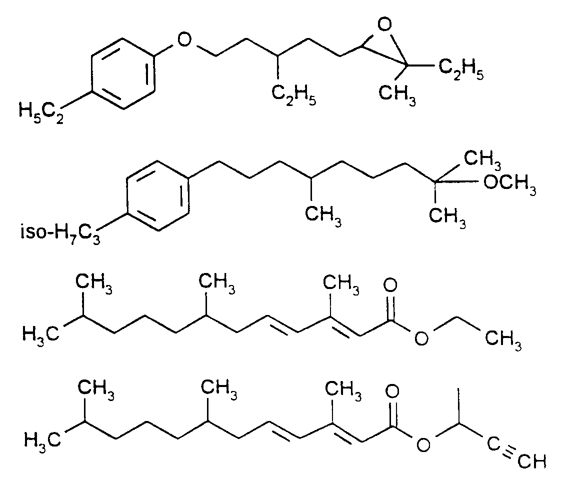

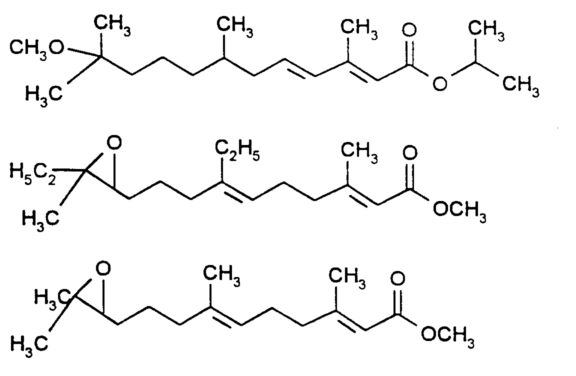

Zu den substituierten Diarylethern gehören insbesondere

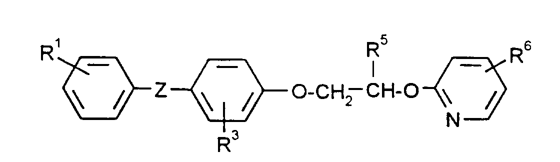

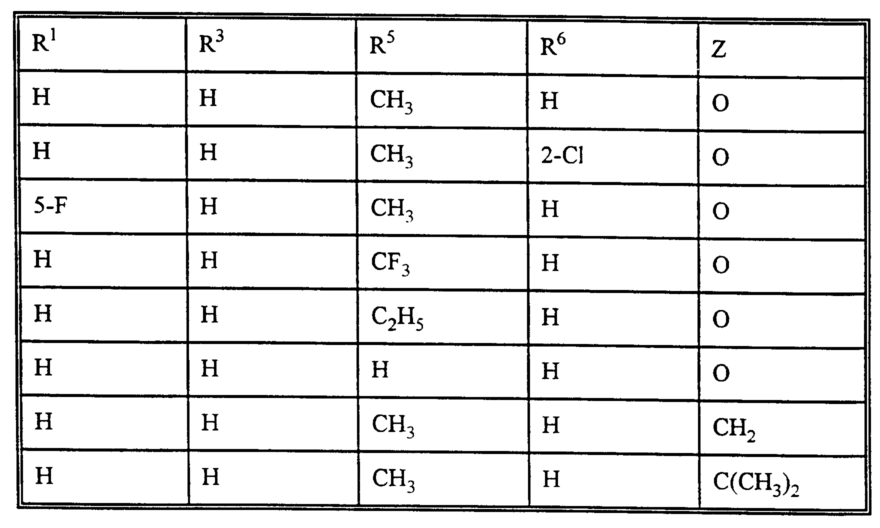

Zu den Benzoylharnstoffen gehören Verbindungen der Formel

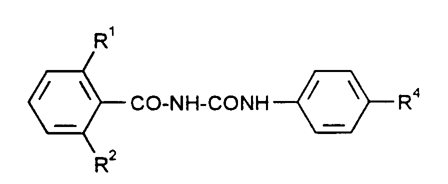

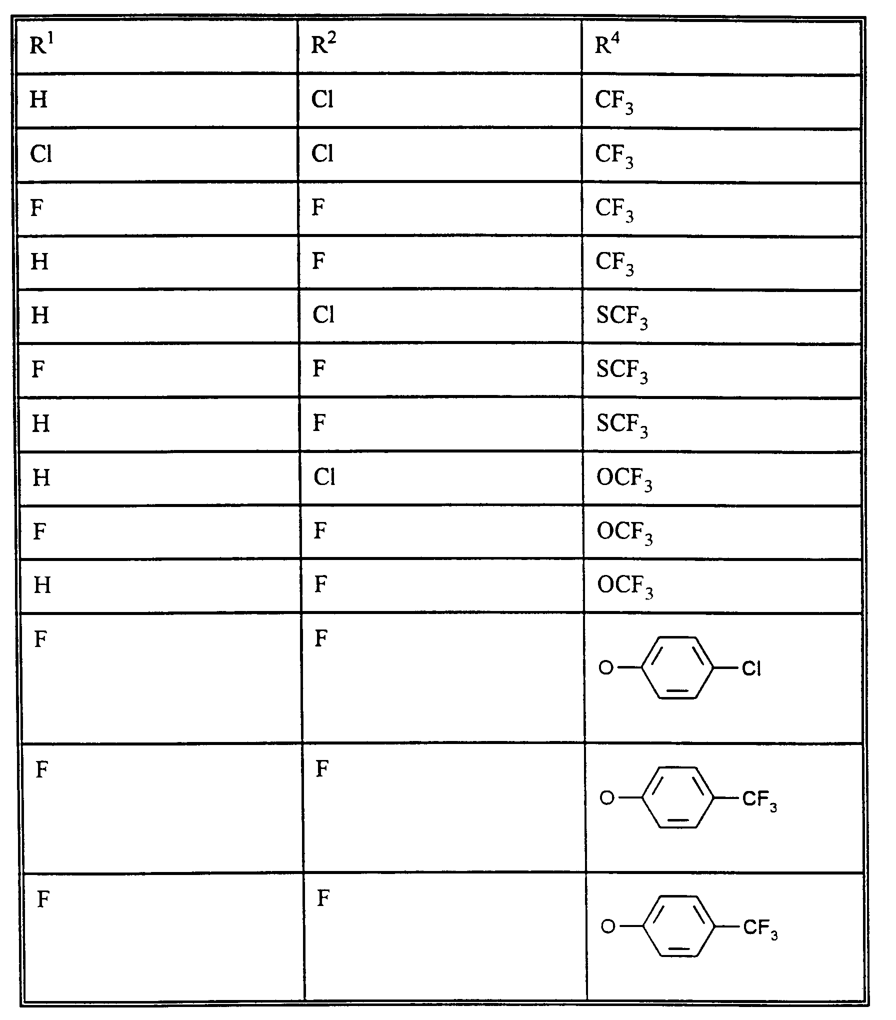

Zu den Triazinen gehören Verbindungen der Formel

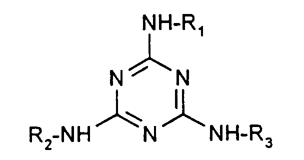

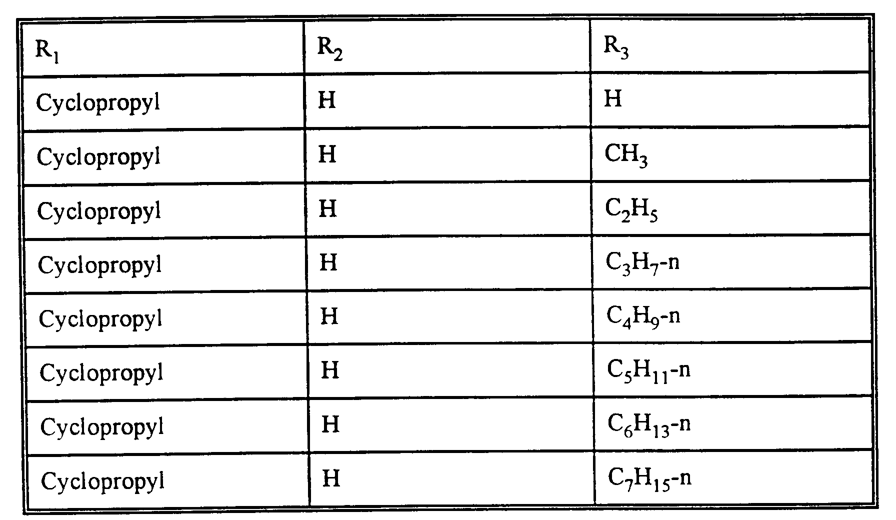

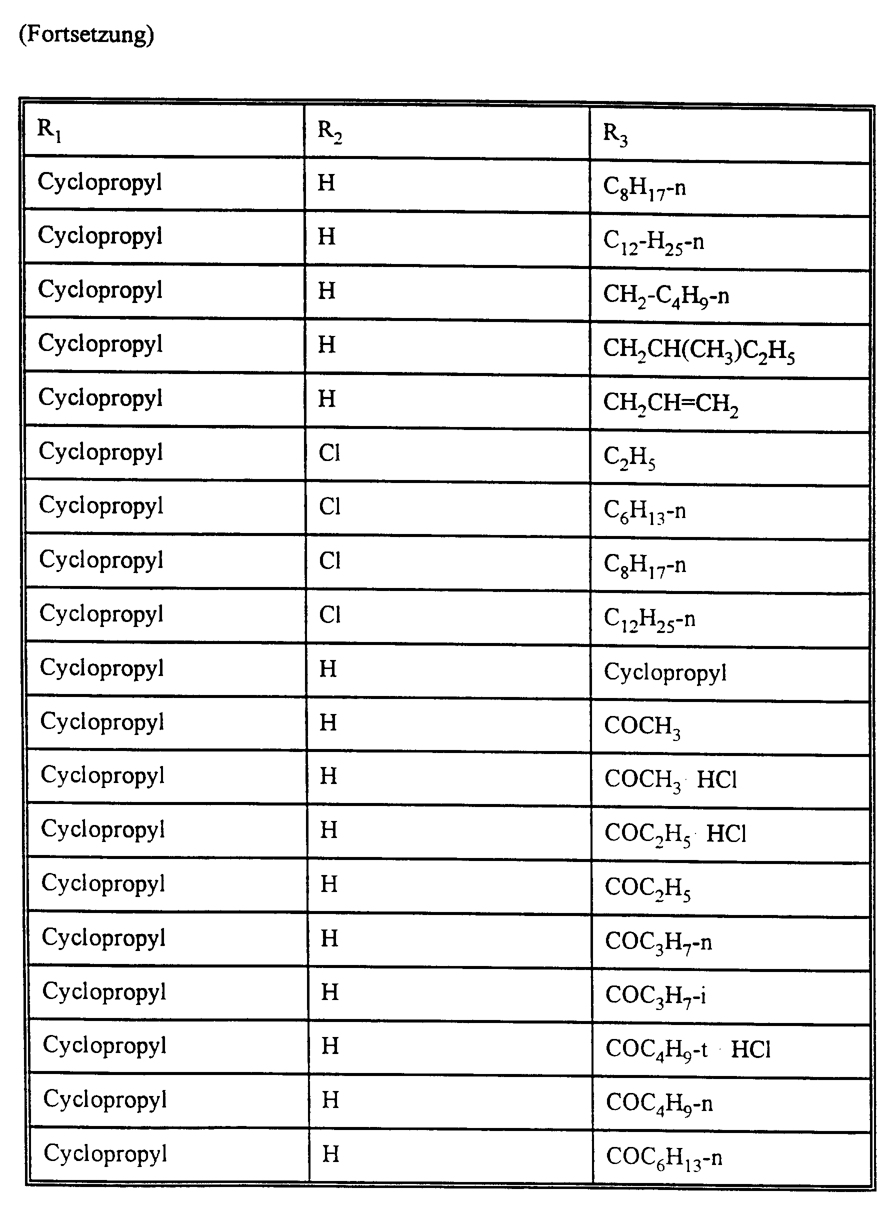

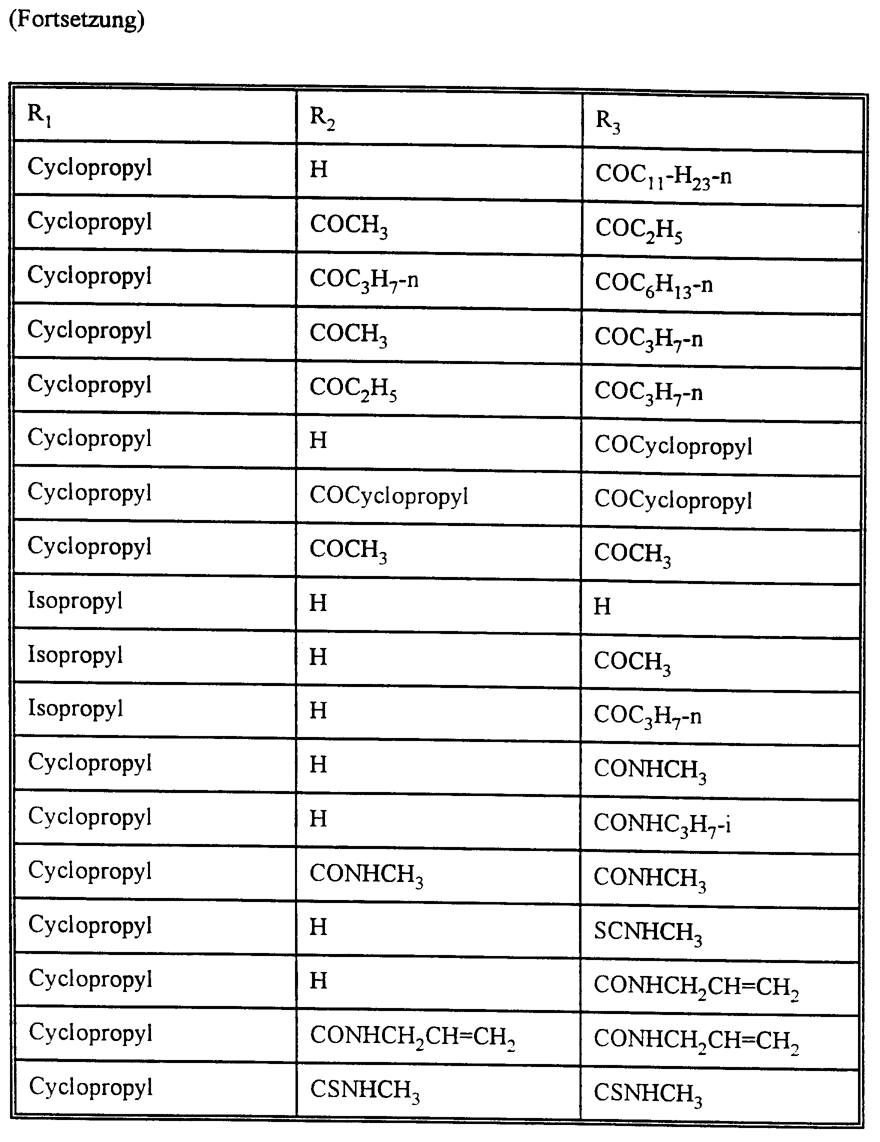

Besonders hervorgehoben seien die weiteren Wirkstoffe mit den common names Propoxur, Cyfluthrin, Flumethrin, Pyriproxyfen, Methoprene, Diazinon, Amitraz, Fenthion und Levamisol.

In den folgenden Beispielen wird als Wirkstoff 1-[(6-Chlor-3-pyridinyl)methyl]-N-nitro-2-imidazolidinium (common name Imidacloprid) eingesetzt.

### Beispiel 1

| | |
|---|---|
| Imidacloprid | 10 g |
| Propylencarbonat | 45 g |
| Benzylalkohol | 45 g |
| ®Belsil DMC 6031 | 1 g |
| (Ein Polysiloxancopolymerisat der Fa. Wacker GmbH, D-81737 München) | |

### Beispiel 2

| | |
|---|---|
| Imidacloprid | 10 g |
| n-Octylpyrrolidon-2 | 44,5 g |
| γ-Butyrolacton | 44,5 g |
| ®Belsil L 066 | 1 g |
| (Ein Polysiloxancopolymerisat der Fa. Wacker GmbH, D-81737 München) | |

### Beispiel 3

| | |
|---|---|
| Imidacloprid | 8,5 g |
| n-Dodecyl-pyrrolidon | 45,25 g |
| γ-Butyrolacton | 45,25 g |
| ®Belsil L 066 | 1 g |
| (Polysiloxancopolymerisat als Spreitmittel) | |

### Beispiel 4

| | |
|---|---|
| Imidacloprid | 10 g |
| Benzylalkohol | 89,9 g |
| ®Belsil DMC 6031 | 0,1 g |
| (Polysiloxancopolymerisat als Spreitmittel) | |

### Beispiel 5

| | |
|---|---|
| Imidacloprid | 12,5 g |
| Benzylalkohol | 70,0 g |
| Propylencarbonat | 17,5 g |

### Beispiel 6

| | |
|---|---|
| Imidacloprid | 10,0 g |
| 1-Cyclohexylpyrrolidon | 80,0 g |
| Propylencarbonat | 10,0 g |

### Beispiel 7

| | |
|---|---|
| Imidacloprid | 11,0 g |
| Benzylalkohol | 70,0 g |
| Propylencarbonat | 15,0 g |
| Isopropylmyristat | 4,0 g |

### Beispiel 8

| | |
|---|---|
| Imidacloprid | 12,5 g |
| Benzylalkohol | 70,0 g |
| Propylencarbonat | 17,4 g |
| Butylhydroxytoluol | 0,1 g |

### Beispiel 9

| | |
|---|---|
| Imidacloprid | 10,0 g |
| Benzylalkohol | 70,0 g |
| Propylencarbonat | 17,5 g |
| Adipinsäure-di-2-ethylhexylester | 2,5 g |

### Beispiel 10

| | |
|---|---|
| Imidacloprid | 12,5 g |
| Pyrrolidon-2 | 70,0 g |
| Propylencarbonat | 17,5 g |

### Beispiel 11

| | |
|---|---|
| Imidacloprid | 10,0 g |
| Pyriproxyfen | 1,0 g |
| Benzylalkohol | 70,0 g |
| Propylencarbonat | 18,9 g |
| Butylhydroxytoluol | 0,1 g |

### Beispiel 12

| | |
|---|---|
| Imidacloprid | 12,5 g |
| Triflumuron | 2,5 g |
| Benzylalkohol | 60,0 g |
| Propylencarbonat | 27,5 g |

### Beispiel 13

| | |
|---|---|
| Imidacloprid | 10,0 g |
| Flumetrin | 2,0 g |
| Benzylalkohol | 60,0 g |
| Propylencarbonat | 28,0 g |

### Beispiel 14

| | |
|---|---|
| Imidacloprid | 10,0 g |
| Benzylalkohol | 60,0 g |
| Ethylencarbonat | 15,0 g |
| Propylencarbonat | 15,0 g |

### Anwendungsbeispiel A

2 ml der in Beispiel 1 beschriebenen Formulierung wurde einem 20 kg schweren Hund der mit Flöhen infestiert war auf den Rücken gegossen. Es wurden folgende Ergebnisse erhalten:

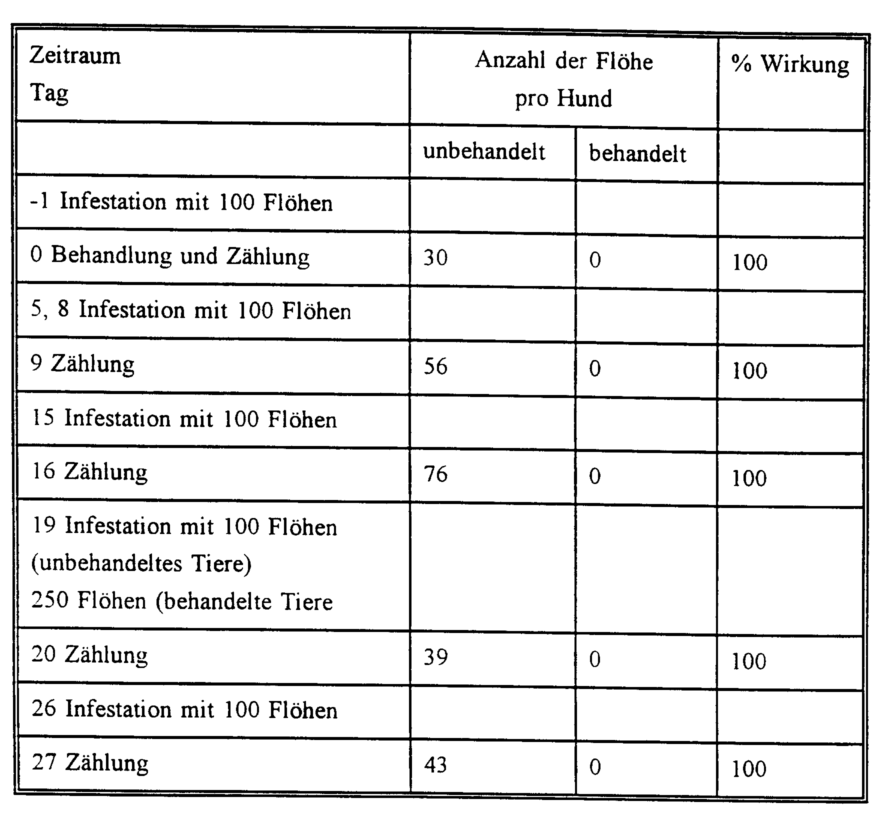

### Anwendungsbeispiel B

1 ml der Lösung gemäß Beispiel 4 wurden auf die Schulter eines 20 kg schweren Hundes gegeben. Das Tier wurde nach 2 und nach 6 Tagen der Behandlung mit 200 Flöhen infestiert. Jeweils am Tag 3 und am Tag 7 nach Behandlung wurden die am Hund verbliebenen Flöhe gezählt. Es konnten keine lebenden Flöhe gefunden werden. Die Wirkung war 100 %.

## Patentansprüche

1. Mittel zur dermalen Bekämpfung von parasitierenden Insekten an Tieren, gekennzeichnet durch einen Gehalt an
- Agonisten oder Antagonisten der nicotinergen Acetylcholinrezeptoren von Insekten in einer Konzentration von 0,1 bis 20 Gew.-% bezogen auf das Gesamtgewicht der Formulierung;
- Lösungsmittel aus der Gruppe Benzylalkohol oder gegebenenfalls substituierte Pyrrolidone in einer Konzentration von mindestens 20 Gew.-% bezogen auf das Gesamtgewicht der Formulierung;
- Lösungsmittel aus der Gruppe cyclischer Carbonate oder Lactone in einer Konzentration von 5,0 bis 50 Gew.-% bezogen auf das Gesamtgewicht der Formulierung;
- gegebenenfalls weitere Hilfsmittel aus der Gruppe Verdickungsmittel, Spreitmittel, Farbstoffe, Antioxidantien, Treibmittel, Konservierungsstoffe, Haftmittel, Emulgatoren, in einer Konzentration von 0,025 bis zu 10 Gew.-% bezogen auf das Gesamtgewicht der Formulierung.

2. Mittel gemäß Anspruch 1, dadurch gekennzeichnet, daß sie als Wirkstoffe Verbindungen der allgemeinen Formel (I) enthalten. 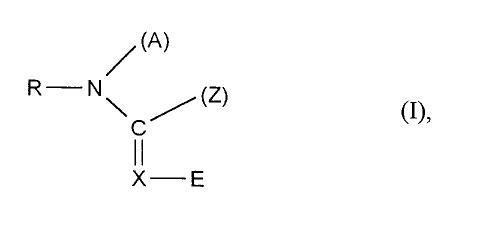 in welcher
R für Wasserstoff, gegebenenfalls substituierte Reste der Gruppe Acyl, Alkyl, Aryl, Aralkyl, Heteroaryl oder Heteroarylalkyl steht;
A für eine monofunktionelle Gruppe aus der Reihe Wasserstoff, Acyl, Alkyl, Aryl steht oder für eine bifunktionelle Gruppe steht, die mit dem Rest Z verknüpft ist;
E für einen elektronenziehenden Rest steht;
X für die Reste -CH= oder =N- steht, wobei der Rest -CH= anstelle eines H-Atoms mit dem Rest Z verknüpft sein kann;
Z für eine monofunktionelle Gruppe aus der Reihe Alkyl, -O-R, -S-R, 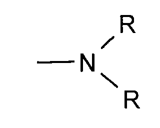 steht, wobei R die oben angegebene Bedeutung hat,
oder für eine bifunktionelle Gruppe steht, die mit dem Rest A oder dem Rest X verknüpft ist.

3. Mittel gemäß Anspruch 1, dadurch gekennzeichnet, daß sie als Wirkstoffe Verbindungen der Formel (I) gemäß Anspruch 2 enthalten, in welcher die Reste folgende Bedeutung haben:
R steht für Wasserstoff sowie für gegebenenfalls substituierte Reste aus der Reihe Acyl, Alkyl, Aryl, Aralkyl, Heteroaryl, Heteroarylalkyl.
Als Acylreste seien genannt Formyl, Alkylcarbonyl, Arylcarbonyl, Alkylsulfonyl, Arylsulfonyl, (Alkyl-)-(Aryl-)-phosphoryl, die ihrerseits substituiert sein können.
Als Alkyl seien genannt C₁₋₁₀-Alkyl, die ihrerseits substituiert sein können.
Als Aryl seien genannt Phenyl, Naphthyl, insbesondere Phenyl.
Als Aralkyl seien genannt Phenylmethyl, Phenethyl.
Als Heteroaryl seien genannt Thienyl, Furyl, Thiazolyl, Imidazolyl, Pyridyl, Benzthiazolyl.
Als Heteroarylalkyl seien genannt Heteroarylmethyl.
Als Substituenten seien aufgeführt:
Alkyl mit 1 bis 4 Kohlenstoffatomen; Alkoxy mit 1 bis 4 Kohlenstoffatomen; Alkylthio mit 1 bis 4 Kohlenstoffatomen; Halogenalkyl mit 1 bis 4 Kohlenstoffatomen und 1 bis 5 Halogenatomen, wobei die Halogenatome gleich oder verschieden sind und als Halogenatome Fluor, Chlor oder Brom stehen; Hydroxy; Halogen; Cyano; Nitro; Amino; Monoalkyl- und Dialkylamino mit 1 bis 4 Kohlenstoffatomen je Alkylgruppe, Carboxyl; Carbalkoxy mit 2 bis 4 Kohlenstoffatomen; Sulfo (-SO₃H); Alkylsulfonyl mit 1 bis 4 Kohlenstoffatomen; Phenylsulfonyl sowie Heteroarylamino und Heteroarylalkylamino.
A steht für Wasserstoff sowie für gegebenenfalls substituierte Reste aus der Reihe Acyl, Alkyl, Aryl, die die bei R angegebenen Bedeutungen haben. A steht ferner für eine bifunktionelle Gruppe. Genannt sei gegebenenfalls substituiertes Alkylen mit 1 bis 4 C-Atomen, wobei als Substituenten die weiter oben aufgezählten Substituenten genannt seien und wobei die Alkylengruppen durch Heteroatome aus der Reihe N, O, S unterbrochen sein können.
A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen Sauerstoff; Schwefel oder Stickstoff und als Heterogruppen N-Alkyl, wobei Alkyl der N-Alkyl-Gruppe 1 oder 2 Kohlenstoffatome enthält. Der heterocyclische Ring enthält 5 oder 6 Ringglieder.
E steht für NO₂, CN.
X steht für -N=
Z steht für gegebenenfalls substituierte Reste Alkyl, -OR, -SR, -NRR, wobei R und die Substituenten die oben angegebene Bedeutung haben.

4. Mittel gemäß Anspruch 1, dadurch gekennzeichnet, daß sie als Wirkstoffe Verbindungen der allgemeinen Formeln (II) und (III) enthalten: 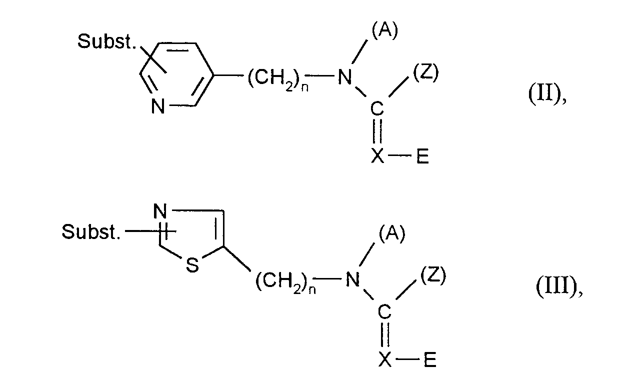 in welchen
n für 1 oder 2 steht,
Subst. für Halogen steht,
A, Z, X und E die in Anspruch 3 angegebenen Bedeutungen haben.

5. Mittel gemäß Anspruch 1, dadurch gekennzeichnet, daß sie als Wirkstoffe Verbindungen der folgenden Formeln enthalten:

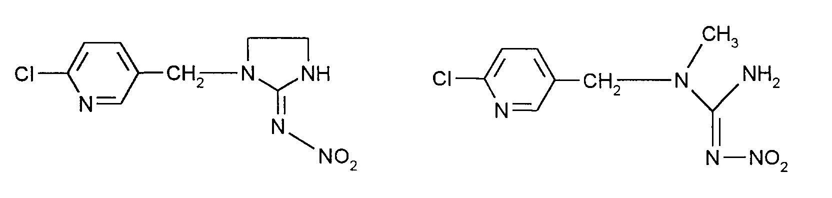

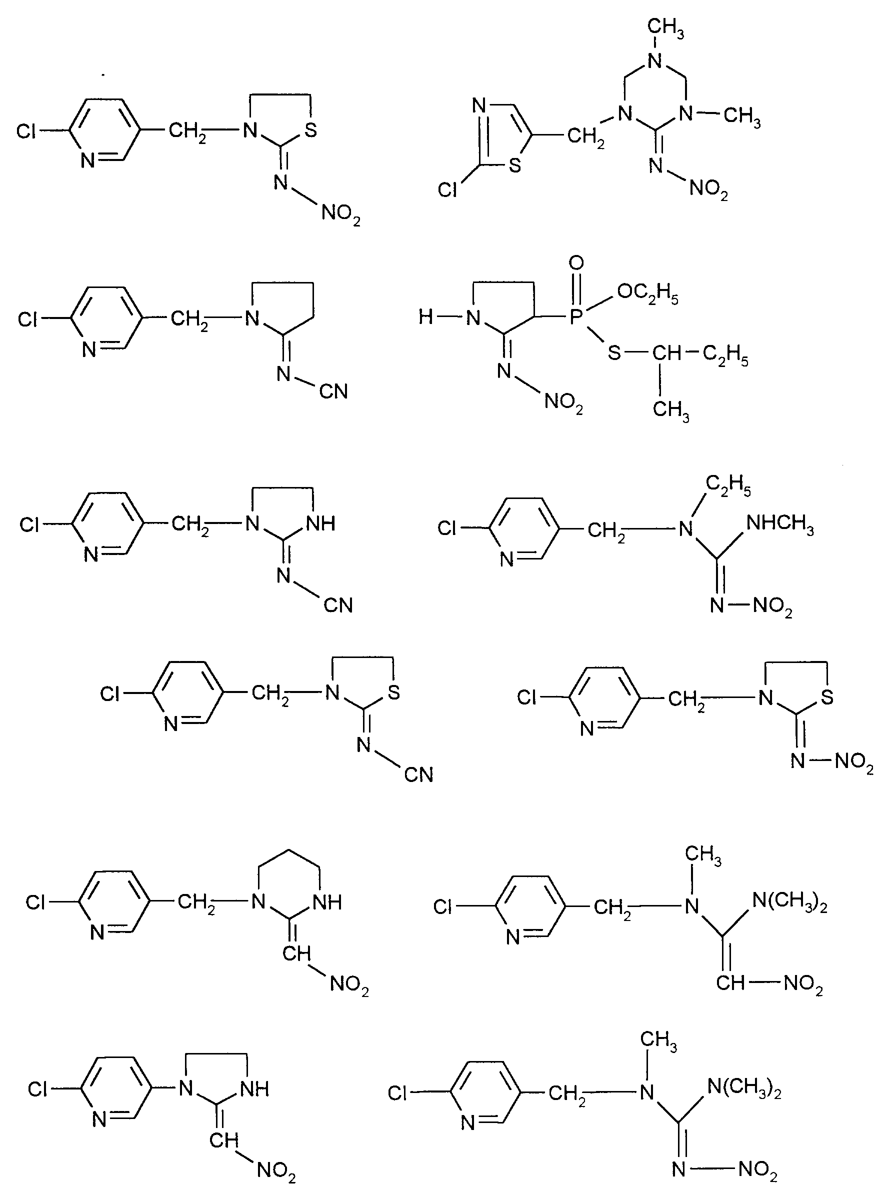

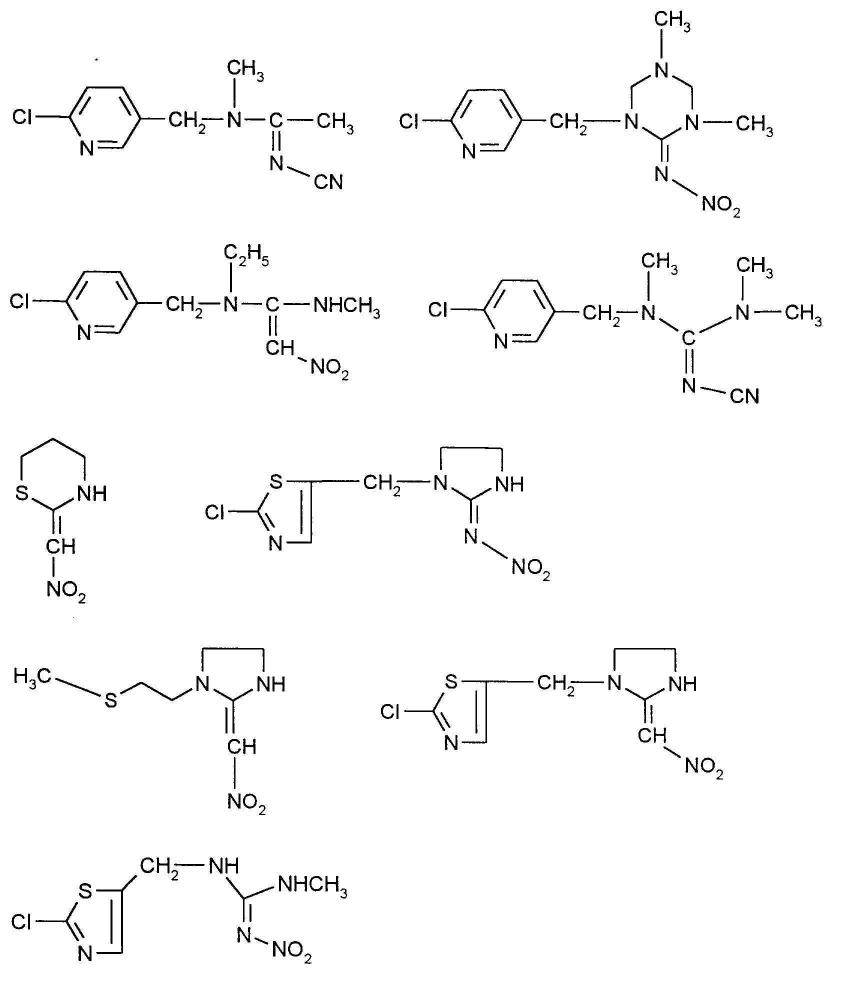

6. Mittel gemäß Anspruch 1, dadurch gekennzeichnet, daß sie als Wirkstoff 1-[(6-Chlor-3-pyridinyl)methyl]-N-nitro-2-imidazolidinimin (common name Imidacloprid) enthalten.

7. Mittel gemäß Ansprüchen 1 bis 6, dadurch gekennzeichnet, daß sie den Wirkstoff in Konzentrationen von 1 bis 12,5 Gew.-% enthalten.

8. Mittel gemäß Ansprüchen 1 bis 7, dadurch gekennzeichnet, daß sie als Lösungsmittel Benzylalkohol enthalten.

9. Mittel gemäß Ansprüchen 1 bis 8, dadurch gekennzeichnet, daß das Lösungsmittel der Gruppe Benzylalkohol oder Pyrrolidone in einer Konzentration von 40 bis 90 Gew.-% vorliegt.

10. Mittel gemäß Ansprüchen 1 bis 9, dadurch gekennzeichnet, daß sie als Lösungsmittel neben dem Lösungsmittel der Gruppe Benzylalkohol oder Pyrrolidone Ethylencarbonat, Propylencarbonat oder γ-Butyrolacton enthalten.

11. Mittel gemäß Ansprüchen 1 bis 10, dadurch gekennzeichnet, daß das Lösungsmittel der Gruppe cyclische Carbonate oder Lactone in einer Konzentration von 7,5 bis 50 Gew.-% vorliegt.

12. Mittel gemäß Ansprüchen 1 bis 11, dadurch gekennzeichnet, daß sie als weitere Wirkstoffe einen Wirkstoff aus der Gruppe Propoxur, Cyfluthrin, Flumethrin, Pyriproxyfen, Methoprene, Diazinon, Amitraz, Fenthion und Levamisol enthalten.

13. Verfahren zur Herstellung der Mittel gemäß Ansprüchen 1 bis 12, dadurch gekennzeichnet, daß man den oder die Wirkstoffe mit den angegebenen Lösungsmitteln vermischt und gegebenenfalls die weiteren Hilfsstoffe zusetzt.

## Claims

1. Compositions for the dermal control of parasitic insects on animals, characterized by a content of
- agonists or antagonists of the nicotinergic acetylcholine receptors of insects in a concentration of from 0.1 to 20% by weight based on the overall weight of the formulation;
- solvents from the group benzyl alcohol or optionally substituted pyrrolidones in a concentration of at least 20% by weight based on the overall weight of the formulation;
- solvents from the group of cyclic carbonates or lactones in a concentration of from 5.0 to 50% by weight based on the overall weight of the formulation;
- if desired, further auxiliaries from the group of thickeners, spreading agents, colorants, antioxidants, propellants, preservatives, adhesives, emulsifiers, in a concentration of from 0.025 to 10% by weight based on the overall weight of the formulation.

2. Compositions according to Claim 1, characterized in that as active substances they comprise compounds of the general formula (I) 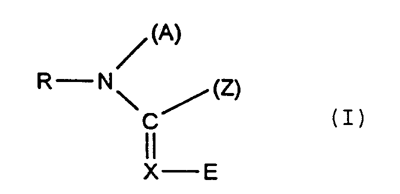 in which
R represents hydrogen or optionally substituted radicals of the group acyl, alkyl, aryl, aralkyl, heteroaryl or heteroarylalkyl;
A represents a monofunctional group from the series hydrogen, acyl, alkyl, aryl or represents a bifunctional group which is linked to the radical Z;
E represents an electron-withdrawing radical;
X represents the radicals -CH= or =N-, it being possible for the radical -CH= to be linked to the radical Z instead of a hydrogen atom;
Z represents a monofunctional group from the series alkyl, -O-R, -S-R, 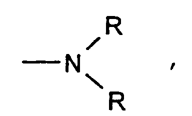 in which R is as defined above, or represents a bifunctional group which is linked to the radical A or to the radical X.

3. Compositions according to Claim 1, characterized in that as active substances they comprise compounds of the formula (I) according to Claim 2 in which the radicals are defined as follows:
R represents hydrogen and represents optionally substituted radicals from the series acyl, alkyl, aryl, aralkyl, heteroaryl, heteroarylalkyl.
As acyl radicals there may be mentioned formyl, alkylcarbonyl, arylcarbonyl, alkylsulphonyl, arylsulphonyl, (alkyl-)-(aryl-)-phosphoryl, which may be substituted in their turn.
As alkyl there may be mentioned C₁₋₁₀-alkyl, which may be substituted in its turn.
As aryl there may be mentioned phenyl, naphthyl, especially phenyl.
As aralkyl there may be mentioned phenylmethyl, phenethyl.
As heteroaryl there may be mentioned thienyl, furyl, thiazolyl, imidazolyl, pyridyl, benzothiazolyl.
As heteroarylalkyl there may be mentioned heteroarylmethyl.
As substituents there may be listed:
alkyl of 1 to 4 carbon atoms; alkoxy of 1 to 4 carbon atoms; alkylthio of 1 to 4 carbon atoms; halogenoalkyl of 1 to 4 carbon atoms and 1 to 5 halogen atoms, the halogen atoms being identical or different and being fluorine, chlorine or bromine; hydroxyl, halogen; cyano; nitro; amino; monoalkyl- and dialkylamino of 1 to 4 carbon atoms per alkyl group, carboxyl; carbalkoxy of 2 to 4 carbon atoms; sulpho (-SO₃H); alkylsulphonyl of 1 to 4 carbon atoms; phenylsulphonyl, and also heteroarylamino and heteroarylalkylamino.
A represents hydrogen and represents optionally substituted radicals from the series acyl, alkyl, aryl, which are as defined for R. A additionally represents a bifunctional group. Mention may be made of optionally substituted alkylene of 1 to 4 carbon atoms, substituents which may be mentioned being those substituents listed above, and the alkylene groups being able to be interrupted by heteroatoms from the series N, O, S.
A and Z can form, together with the atoms to which they are attached, a saturated or unsaturated heterocyclic ring. The heterocyclic ring can contain a further 1 or 2 identical or different heteroatoms and/or hetero-groups. Heteroatoms are oxygen, sulphur or nitrogen, and hetero-groups are N-alkyl, where alkyl in the N-alkyl group contains 1 or 2 carbon atoms. The heterocyclic ring contains 5 or 6 ring members.
E represents NO₂, CN.
X represents -N=.
Z represents optionally substituted radicals alkyl, -OR, -SR, -NRR, where R and the substituents are as defined above.

4. Compositions according to Claim 1, characterized in that as active substances they comprise compounds of the general formulae (II) and (III): 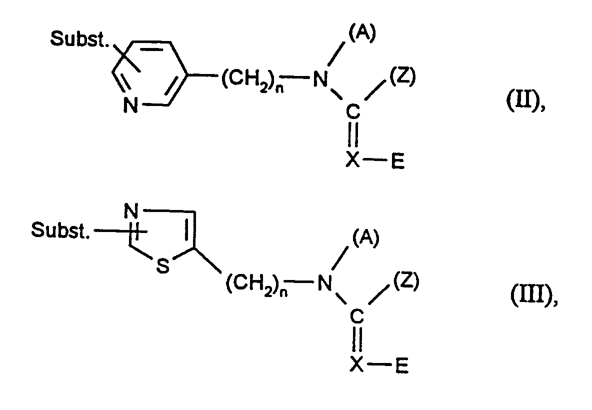 in which
n represents 1 or 2,
Subst. represents halogen,
A, Z, X and E are as defined in Claim 3.

5. Compositions according to Claim 1, characterized in that as active substances they comprise compounds of the following formulae:

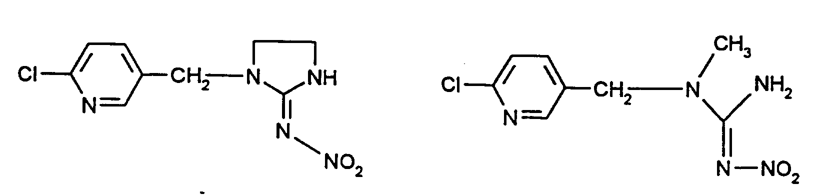

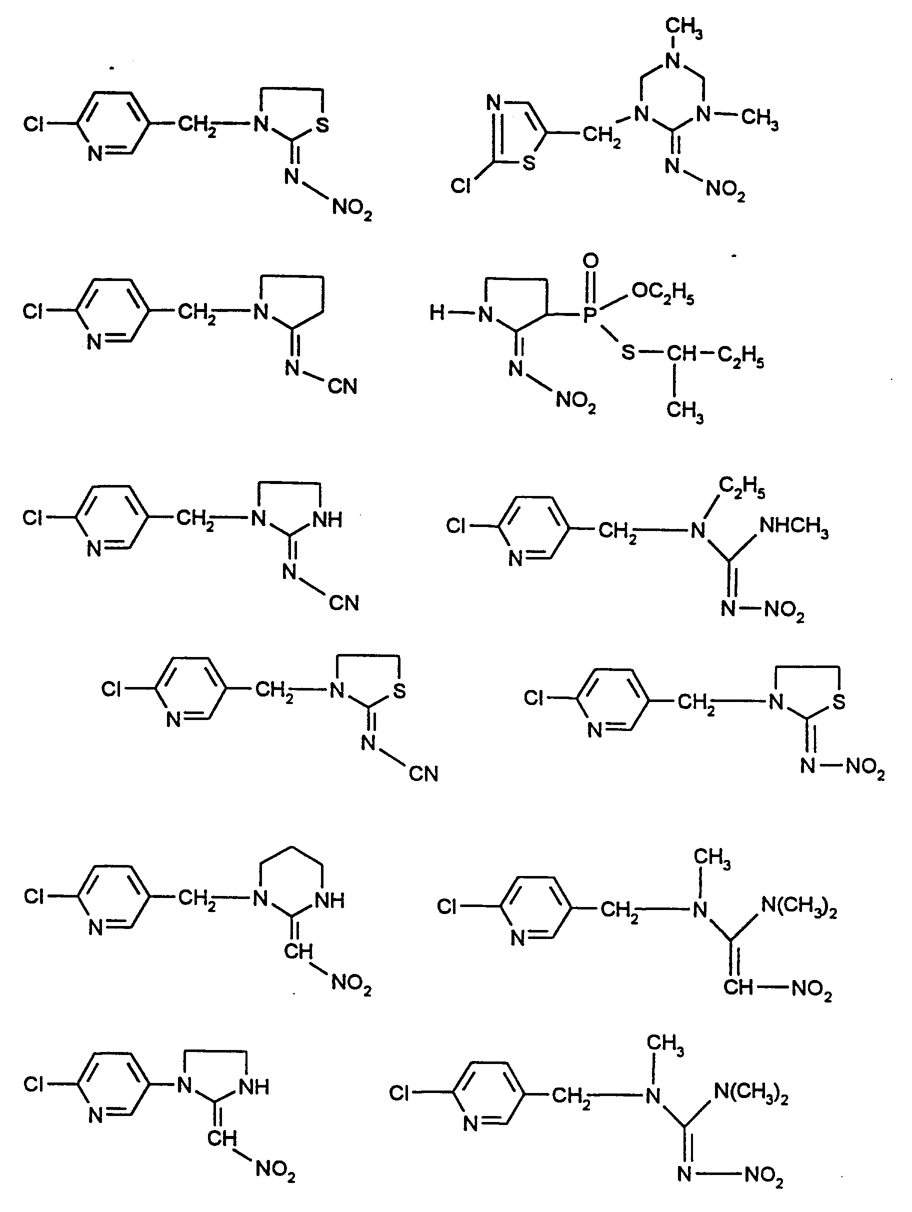

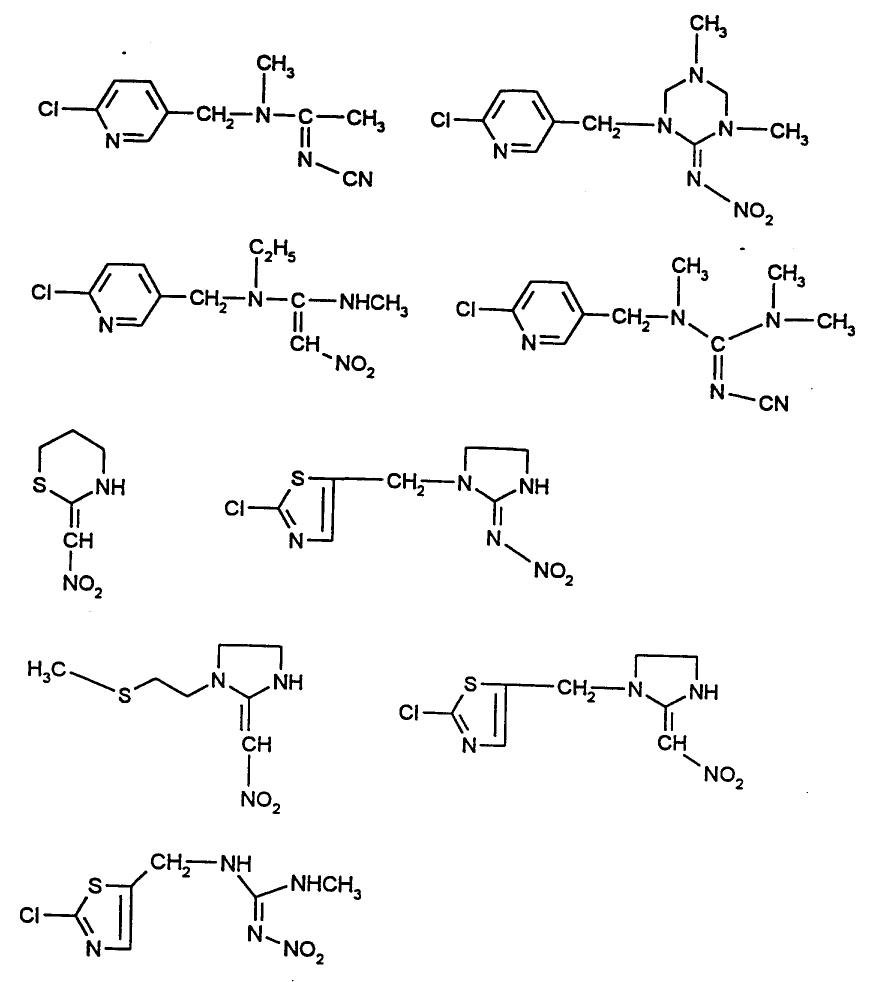

6. Compositions according to Claim 1, characterized in that as active substance they comprise 1-[(6-chloro-3-pyridinyl)methyl]-N-nitro-2-imidazolidinimine (common name imidacloprid).

7. Compositions according to Claims 1 to 6, characterized in that they comprise the active substance in concentrations of from 1 to 12.5% by weight.

8. Compositions according to Claims 1 to 7, characterized in that they comprise benzyl alcohol as solvent.

9. Compositions according to Claims 1 to 8, characterized in that the solvent of the group benzyl alcohol or pyrrolidone is present in a concentration of from 40 to 90% by weight.

10. Compositions according to Claims 1 to 9, characterized in that as solvents they comprise, in addition to the solvent of the group benzyl alcohol or pyrrolidones, ethylene carbonate, propylene carbonate or γ-butyrolactone.

11. Compositions according to Claims 1 to 10, characterized in that the solvent of the group cyclic carbonates or lactones is present in a concentration of from 7.5 to 50% by weight.

12. Compositions according to Claims 1 to 11, characterized in that as further active substances they comprise an active substance from the group propoxur, cyfluthrin, flumethrin, pyriproxyfen, methoprene, diazinon, amitraz, fenthion and levamisol.

13. Process for producing the compositions according to Claims 1 to 12, characterized in that the active substance or substances is or are mixed with the stated solvents and the further auxiliaries are added if desired.

## Revendications

1. Agents pour la lutte par voie dermique contre des insectes parasitaires sur des animaux, caractérisés en ce qu'ils contiennent
des agonistes ou des antagonistes des récepteurs nicotinergiques de l'acétylcholine d'insectes en une concentration de 0,1 à 20% en poids rapportés au poids total de la formulation;
des solvants choisis parmi le groupe comprenant l'alcool benzylique ou des pyrrolidones éventuellement substituées, en une concentration d'au moins 20% en poids rapportés au poids total de la formulation;
des solvants choisis parmi le groupe comprenant des carbonates ou des lactones cycliques en une concentration de 5,0 à 50% en poids rapportés au poids total de la formulation;
le cas échéant, d'autre adjuvants choisis parmi le groupe comprenant des épaississants, des agents d'étalement, des colorants, des antioxydants, des agents moussants, des conservants, des adhésifs, des émulsifiants, en une concentration de 0,025 à 10% en poids rapportés au poids total de la formulation.

2. Agents selon la revendication 1, caractérisés en ce qu'ils contiennent, à titre de substances actives, des composés répondant à la formule générale (I) dans laquelle 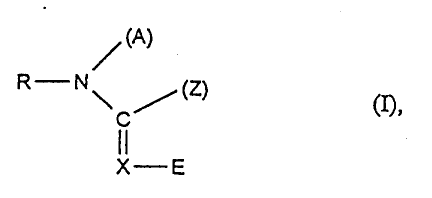
R représente un atome d'hydrogène, des radicaux éventuellement substitués choisis parmi le groupe comprenant un radical acyle, un radical alkyle, un radical aryle, un radical aralkyle, un radical hétéroaryle ou un radical hétéroarylakyle;
A représente un groupe monofonctionnel choisi parmi la série comprenant un atome d'hydrogène, un groupe acyle, un groupe alkyle, un groupe aryle, ou bien un groupe bifonctionnel qui est lié au radical Z;
E représente un radical attirant les électrons;
X représente les radicaux -CH= ou =N-, le radical -CH=, au lieu de comporter un atome de H, pouvant être lié au radical Z;
Z représente un groupe monofonctionnel choisi parmi la série comprenant un groupe alkyle, un groupe -O-R, un groupe -S-R, un groupe 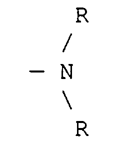 où R possède la signification indiquée ci-dessus,
ou encore représente un groupe bifonctionnel qui est lié au radical A ou au radical X.

3. Agents selon la revendication 1, caractérisés en ce qu'ils contiennent à titre de substances actives des composés de formule (I) selon la revendication 2, dans lesquels les radicaux ont la signification ci-après:
R représente un atome d'hydrogène ou encore des radicaux éventuellement substitués choisis parmi la série comprenant un groupe acyle, un groupe alkyle, un groupe aryle, une groupe aralkyle, un groupe hétéroaryle, un groupe hétéroarylakyle.
A titre de radicaux acyle, on mentionnera un
groupe formyle, un groupe alkylcarbonyle, un groupe arylcarbonyle, un groupe alkylsulfonyle, un groupe arylsulfonylyle, un groupe (alkyl-)-(aryl-)phosphoryle, qui pour leur part peuvent être substitués.
A titre de groupe alkyle, on mentionnera un groupe alkyle en C₁-C₁₀ qui pour sa part peut être substitué.
A titre de groupe aryle, on mentionnera un groupe phényle, un groupe naphtyle, en particulier un groupe phényle.
A titre de groupe aralkyle, on mentionnera un groupe phénylméthyle, un groupe phénéthyle.
A titre de radical hétéroaryle, on mentionnera un groupe thiényle, un groupe furyle, un groupe thiazolyle, un groupe imidazolyle, un groupe pyridyle, un groupe benzthiazolyle.
A titre de radical hétéroarylalkyle, on mentionnera un groupe hétéroarylméthyle.
A titre de substituants, il y a lieu de mentionner:
un groupe alkyle contenant de 1 à 4 atomes de carbone; un groupe alkoxy contenant de 1 à 4 atomes de carbone; un groupe alkylthio contenant de 1 à 4 atomes de carbone; un groupe halogénalkyle contenant de 1 à 4 atomes de carbone et de 1 à 5 atomes d'halogène, les atomes d'halogène étant identiques ou différents et représentant un atome de fluor, un atome de chlore ou un atome de brome; un groupe hydroxyle; un atome d'halogène; un groupe cyano; un groupe nitro; un groupe amino; un groupe monoalkyl- et dialkylamino contenant de 1 à 4 atomes de carbone par groupe alkyle; un groupe carboxyle; un groupe carbalcoxy contenant de 2 à 4 atomes de carbone; un groupe sulfo (-SO₃H); un groupe alkylsulfonyle contenant de 1 à 4 atomes de carbone; un groupe phénylsulfonyle; ainsi qu'un groupe hétéroarylamino et un groupe hétéroalkylarylamino.
A représente un atome d'hydrogène, ainsi que des radicaux éventuellement substitués choisis parmi la série comprenant un groupe acyle, un groupe alkyle, un groupe aryle, qui ont les significations indiquées pour R. A représente en outre un groupe bifonctionnel. On mentionnera un groupe alkylène éventuellement substitué contenant de 1 à 4 atomes de carbone; à titre de substituants, on mentionnera les substituants énumérés ci-dessus et les groupes alkylènes pouvant être interrompus par des hétéroatomes choisis parmi la série comprenant un atome d'azote, un atome d'oxygène et un atome de soufre.
A et Z peuvent former, de manière conjointe avec les atomes auxquels ils sont liés, un noyau hétérocyclique saturé ou insaturé. Le noyau hétérocyclique peut contenir 1 ou 2 hétéroatomes et/ou hétérogroupes identiques ou différents. A titre d'hétéroatomes, on mentionnera un atome d'oxygène, un atome de soufre ou un atome d'azote, et à titre d'hétérogroupes, on mentionnera un groupe N-alkyle, le radical alkyle du groupe N-alkyle contenant 1 ou 2 atomes de carbone. Le noyau hétérocyclique contient 5 ou 6 chaînons.
E représente un groupe NO₂, un groupe CN.
X représente -N=
Z représente des radicaux alkyle, un groupe -OR, un groupe -SR, un groupe -NRR éventuellement substitués, R et les substituants possédant la signification indiquée ci-dessus.

4. Agents selon la revendication 1, caractérisés en ce qu'ils contiennent à titre de substances actives des composés répondant aux formules générales (II) et (III): 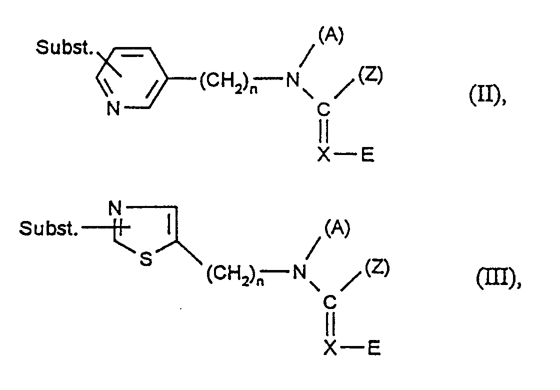 dans lesquelles
n représente 1 ou 2
Subst. représente un atome d'halogène,
A, Z, X et E ont les significations indiquées à la revendication 3.

5. Agents selon la revendication 1,caractérisés en ce qu'ils contiennent à titre de substances actives des composés répondant aux formules ci-après:

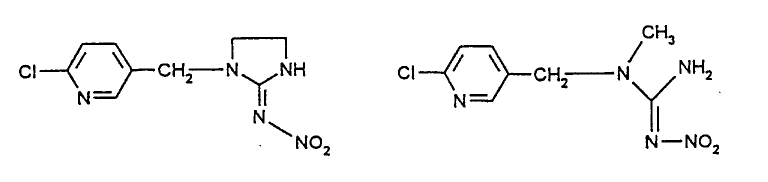

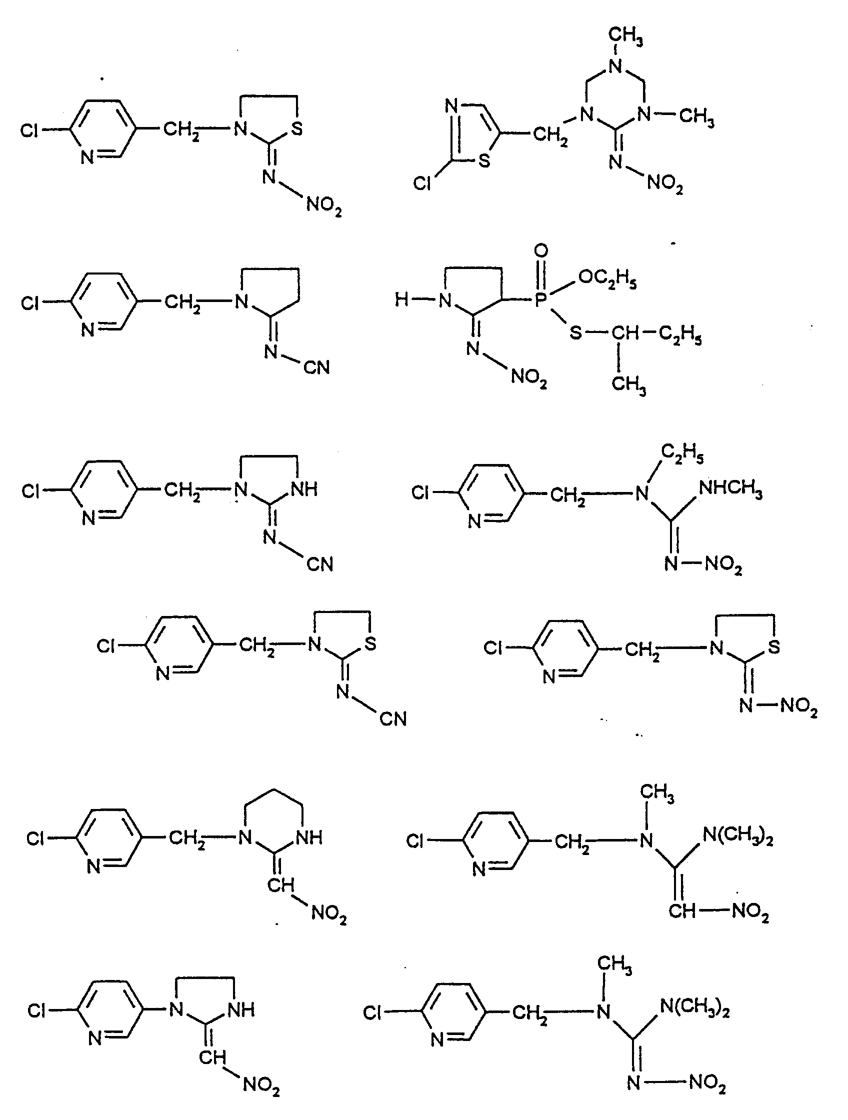

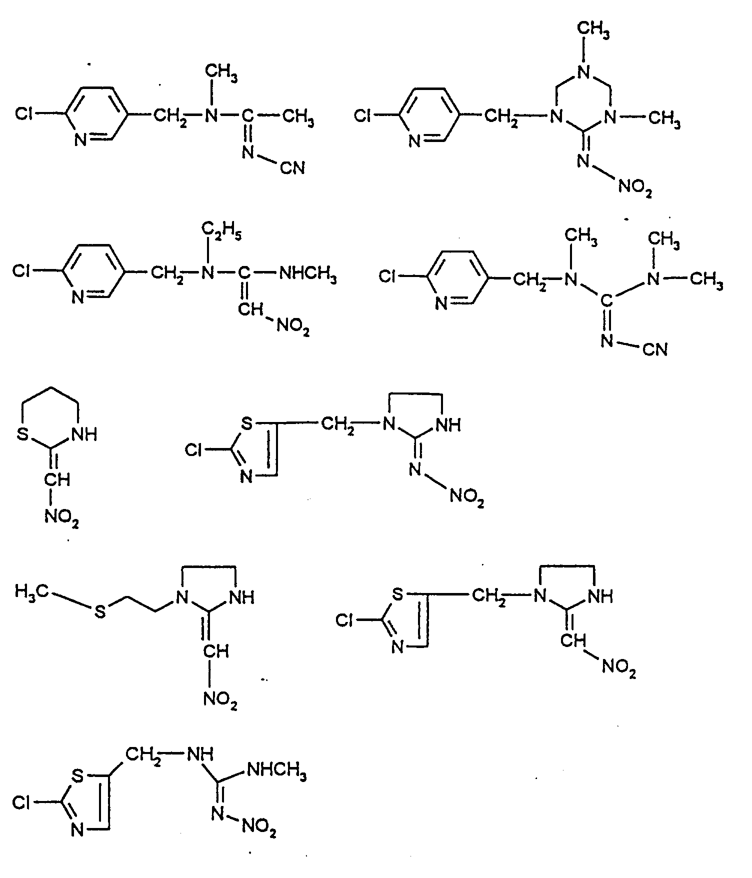

6. Agents selon la revendication 1, caractérisés en ce qu'ils contiennent à titre de substance active, la 1-[(6-chloro-3-pyridinyl)méthyl]-N-nitro-2-imidazolidinimine (dénomination commune Imidacloprid).

7. Agents selon les revendications 1 à 6, caractérisés en ce qu'ils contiennent la substance active dans des concentrations de 1 à 12,5% en poids.

8. Agents selon les revendications 1 à 7, caractérisés en ce qu'ils contiennent de l'alcool benzylique à titre de solvant.

9. Agents selon les revendications 1 à 8, caractérisés en ce que le solvant choisi parmi le groupe comprenant l'alcool benzylique ou des pyrrolidones est présent en une concentration de 40 à 90% en poids.

10. Agents selon les revendications 1 à 9, caractérisés en ce qu'ils contiennent, outre le solvant choisi parmi le groupe comprenant l'alcool benzylique ou des pyrrolidones, le carbonate d'éthylène, le carbonate de propylène ou la γ-butyrolactone.

11. Agents selon les revendications 1 à 10, caractérisés en ce que le solvant choisi parmi le groupe comprenant des carbonates ou des lactones cycliques, est présent en une concentration de 7,5 à 50% en poids.

12. Agents selon les revendications 1 à 11, caractérisés en ce qu'ils contiennent, à titre de substances actives supplémentaires, une substance active choisie parmi le groupe comprenant Propoxur, Cyfluthrin, Flumethrin, Pyriproxyfen, Methoprene, Diazinon, Amitraz, Fenthion et Levamisol.

13. Procédé pour la préparation des agents selon les revendications 1 à 12, caractérisé en ce qu'on mélange la ou les substances actives avec les solvants indiqués et on ajoute, le cas échéant, les adjuvants supplémentaires.
